# EUROPEAN PATENT APPLICATION

(11) **EP 1 800 619 A1**
(43) Date of publication of application: **27.06.2007**
(21) Application number: 05785764.1
(22) Date of filing: 26.07.2005
(51) Int. Cl.: A61F 2/02

(54) **MESH FOR THE SURGICAL TREATMENT OF URINARY INCONTINENCE**

(30) Priority: 06.10.2004 ES 200402270 U
(71) Applicant: Romero Maroto, Jesus, 03560 El Campello (Alicante) (ES)
(72) Inventor: Romero Maroto, Jesus, 03560 El Campello (Alicante) (ES)
(74) Representative: SUGRANES - VERDONCES - FERREGÜELA
(86) International application number: PCT/ES2005/000419
(87) International publication number: WO 2006/040366

(57) **Abstract**

The invention relates to a mesh for the surgical treatment of urinary incontinence, specifically stress incontinence, of the type that takes the form of a considerably-long rectangular strip, the ends of which narrow and converge such as to terminate in filiform elements. The invention comprises the mesh (1) which takes the form of a considerably-long strip and which, in the preferred embodiment thereof, adopts a roughly rectangular shape. According to the invention, the ends of the mesh narrow and converge such as to terminate in filiform elements (2). The strips can be provided with suitably positioned holes (3), such as in the examples represented in figures 1 y 2, or may contain no holes, as in the case of figure 3. The aforementioned holes (3) are grouped together in pairs which form transverse lines, as in figure 1, or longitudinal lines as in figure 2.

## Description

### Object of the invention

The present invention relates to a mesh that can be used in surgical operations for the treatment of stress urinary incontinence, which is designed to return the bladder neck and the urethra to their original correct position and which is implanted with the aid of a mesh-passing device.

The object of the invention is to provide said mesh with a set of threads, the ends of which are situated outside the cutaneous vaginal suture lines once the intervention is finished so that, by pulling on said threads, it is possible to tighten/loosen the mesh in order to eliminate any obstruction or correct incontinence, should it persist.

### Background of the invention

Stress urinary incontinence is generally known to be a common pathology in women, consisting of the loss of urine when an abdominal effort is made, e.g. on coughing, moving from a sitting to a standing position or walking.

The aforementioned problem is a condition that often results in a significant reduction in quality of life as a consequence of the voluntary limitation of many activities due to fear and embarrassment that other people may notice said loss, and that can sometimes leads to situations of loneliness and isolation.

The anatomical alteration underlying this urine loss symptomatology is a weakened support of the bladder neck and the urethra as a consequence of an alteration in the perineal muscles caused, among other reasons, by childbearing. This weakened support of the bladder neck and the urethra means that abdominal pressure is not correctly transmitted thereto, this altered pressure being the cause of involuntary leakages of urine.

So far several techniques have been proposed that provide solutions to this problem. In any case, the solution to said problem of incontinence is surgical and consists of returning the bladder neck and the urethra to their original situation, then providing them with a support that enables the correct transmission of abdominal pressure thereto. Of the techniques used, that known as the "sling" technique provides the most effective and lasting solution.

This sling technique consists of placing a strip of tissue, either from the patient herself or heterologous tissue (marlex and silicone have been used, as has polypropylene with better results), in a sub-cervical or sub-urethral position so that, once said band has been attached at different points depending on the technique, it raises and supports the bladder neck and the urethra in their original position, thus enabling the transmission of pressure and preventing urine loss.

The strip and of tissue can be long enough to stretch from the urethra to the hypogastrium, or it can simply consist of a small patch joined to four threads, which are what subsequently fasten it to the abdominal wall.

The aggressiveness of the technique has decreased significantly since it has been performed transvaginally without the need to open the pelvic cavity and since polypropylene has been used, as it avoids the need to take aponeuroses from the patient herself.

Recently, within the concept of this surgery as a minimally invasive approach, two systems have come onto the market for surgery to treat urinary incontinence, under the initials TVT and SPARC. Both comprise a polypropylene mesh covered by a plastic sheath and a connecting system consisting of a needle with a handle. The two techniques differ in that, in the case of TVT, the needle is passed from the vagina towards the hypogastrium, which involves separating the urethra using a catheter with a stiffening element, whereas in the case of the SPARC technique it is passed from the hypogastrium towards the vagina without the need to separate the urethra using a catheter. In both cases, the polypropylene mesh used, which is covered in a plastic sheath, is passed from the vagina to the hypogastrium. Once the mesh has been passed through, the plastic sheath is removed and extracted, having aided the passage of the mesh and leaving it situated in a subcutaneous position, where it is secured thanks to the properties with which it is provided.

The greatest problem presented with these techniques and all those that use the sling system is the appropriate regulation of the tension of the sling, a factor that determines the success or failure of this type of surgery. On the one hand, if subjected to excessive tension it can cause a urinary obstruction, thereby causing the patient difficulties in voiding urine. On the other hand, if insufficient tension is provided, the patient will continue to present problems of incontinence.

There is therefore a practical difficulty associated with the fact that there is no way of calculating the most suitable tension, it being necessary to do this randomly and approximately. Once the operation has finished, it is no longer possible to correct the excess or lack of tension, which leads to an estimated 10% to 20% failure rate of these interventions. Furthermore, this problem is the cause of all failures that occur with the application of this technique.

The existence on the market of a system called REEMEX is also known, which is used to perform the subcutaneous implantation of a pulley-like device in the hypogastrium, making it possible, with the use of a screwdriver in the postoperative period, to increase the tension of the sling formed by a small patch of tissue held by four threads. This system is expensive, complex and is prone to considerable potential complications due to the fact that it requires the implantation of a foreign body. It also enables the sling to be loosened if it has been secured using threads, but it does not allow the system to be used when a sling made of the mesh alone, i.e. of the TVT, SPARC or TOT type, is used.

Lastly, another system that currently exists but is not known to be on the market is that developed by Dr Gil Vemet, which includes a fluid reservoir implanted in the subcutaneous tissue in the hypogastrium. The threads that secure the sling are attached to said reservoir. The system makes it possible to fill the fluid reservoir if there is not enough tension, thus increasing the tension. The clinical application of this system is also difficult and it cannot be used with complete meshes.

Another technique that has recently appeared is TOT, which differs from the previous techniques in that the mesh is passed through the obturator foramen instead of the hypogastrium, with the same intention of returning the urethra and the bladder neck to their correct position. This technique presents the same difficulty of adjusting the exact tension of the mesh, which also results in a significant failure rate.

The applicant is the owner of the Spanish patent of invention with application number P 200201227, which discloses a system for the treatment of stress urinary incontinence that solves the problems of the above-described techniques, providing an effective, quick and economical solution, particularly with regard to the application of the sling system.

This patent uses a mesh passer or thread-passing needle and the mesh itself, which is designed for application in maintaining the bladder neck and the urethra in an optimum position. This mesh does not have any covering and it also presents a series of holes suitably distributed in specific positions thereon, which are positioned once the mesh has been placed at the aponeurosis of the rectus and periurethral level, allowing threads to be inserted into said holes whose ends are situated outside the vaginal and cutaneous suture lines once the intervention has finished, so that by pulling on the vaginal or cutaneous threads it is possible to tighten or loosen them, respectively, in order to eliminate any obstruction or correct persisting incontinence, respectively. This can be done the day after the intervention, with the patient awake, going about her normal activities and thus making it possible to correct any dysfunction deriving from the implantation of the sling.

### Description of the invention

The mesh proposed by the invention, which is designed for use in the system of said patent of invention P 200201227, considerably facilitates its use by the surgeon.

For this and more specifically, the characteristics of said mesh, which can either be provided with the aforementioned holes or not and can either be covered with a sheath to aid its passage through the tissues, are based on the fact that it includes vaginal and cutaneous threads either inserted into said holes, should there be any, or inserted through the gaps in the mesh when there are no holes, in equivalent positions so that, thanks to said threads, the surgeon does not need to perform the operation of attaching them to the mesh.

### Description of the drawings

To complement this description and in order to aid comprehension of the invention's characteristics, according to a preferred practical embodiment thereof, an illustrative and non-limiting set of drawings is provided an integral part of said description. These are as follows:
- Figure 1: shows a schematic plan view drawing of a mesh for the surgical treatment of stress urinary incontinence in an embodiment according to the object of the present invention, specifically according to a first variant wherein said mesh is provided with holes.
- Figure 2: shows a similar drawing to the previous figure, but with a different arrangement of said holes.
- Figure 3: lastly, shows a similar drawing to the previous figures, but wherein the mesh itself does not have holes through which to pass the threads.

### Preferred embodiment of the invention

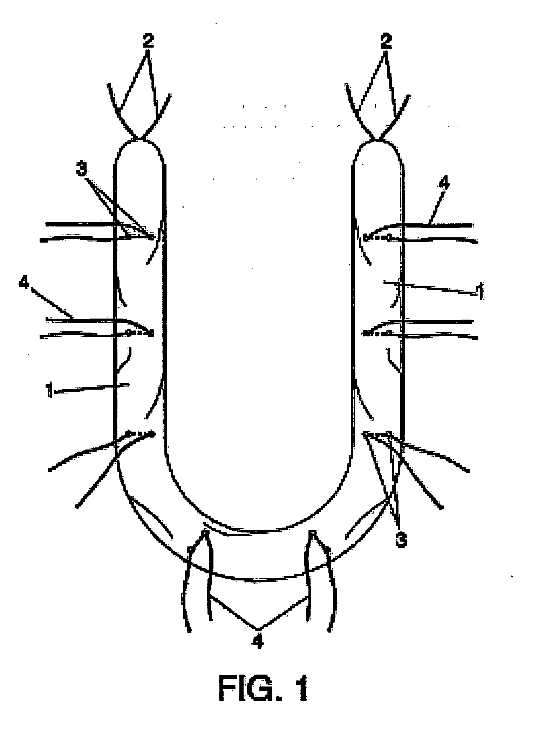

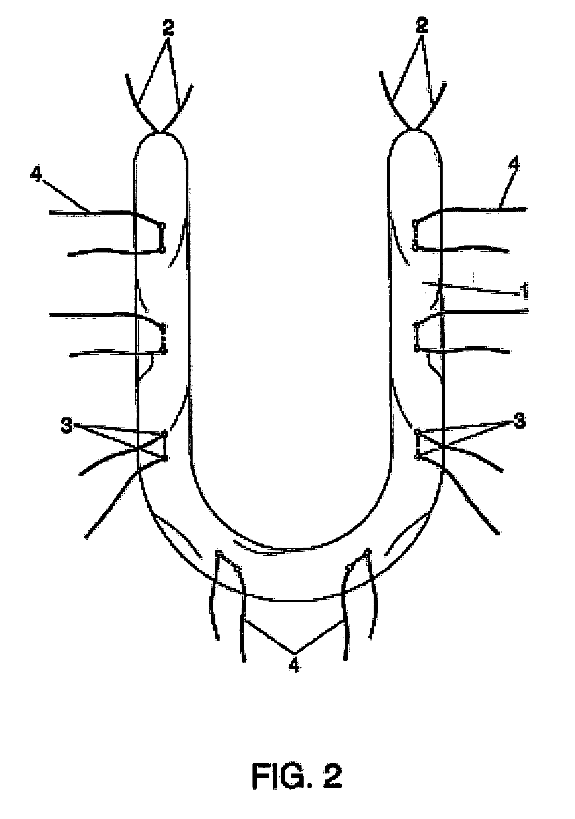

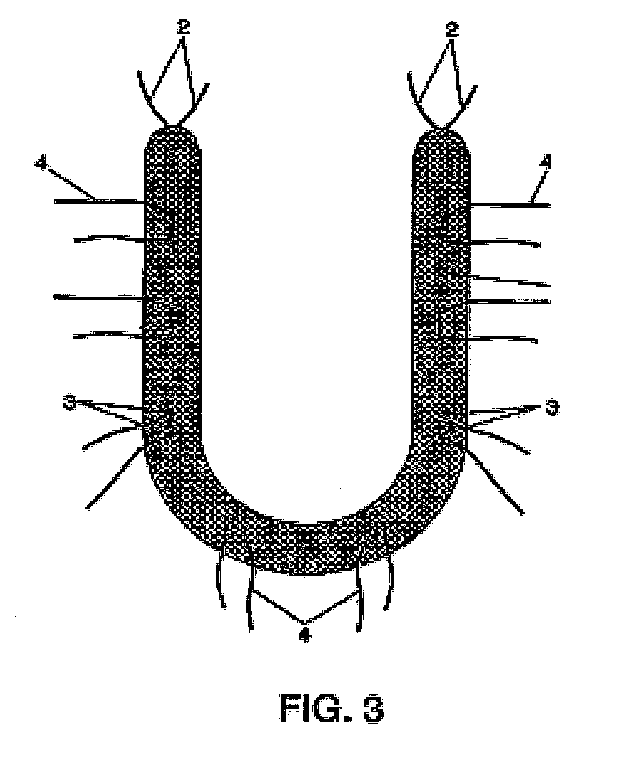

In view of the above-described figures it can be observed how the intervention proposed by the invention is made, in all cases, using a mesh itself (1) in the form of a substantially elongated strip, which, in the preferred embodiment thereof adopts a roughly rectangular shape, the ends of which narrow and converge to terminate in filiform elements (2). The strips can be provided with suitably positioned holes (3), as in the examples shown in figures 1 and 2, or may contain no holes, as in figure 3. The aforementioned holes (3) are grouped together in pairs to form transverse lines, as in figure 1, or longitudinal lines, as in figure 2.

According to the invention and according to the variants shown in figures 1 and 2, a thread (4) is passed through each of the pairs of holes (3), whereas in the case of figure 3 the threads (4) are passed through the gaps in the mesh itself (1) in equivalent positions to those of one of the two previous figures, so that these threads (4) are available, i.e. previously attached to the mesh, without this operation having to be performed immediately prior to the surgical intervention.

These threads (4) will finally be situated outside the vaginal and cutaneous suture lines so that, as has been said before, by pulling on them it is possible to tighten/loosen the mesh in order to eliminate or correct any problem of obstruction or incontinence, should it persist.

## Claims

1. Mesh for the surgical treatment of urinary incontinence, specifically stress incontinence, of the type that takes the form of a considerably elongated rectangular strip, the ends of which narrow and converge to terminate in filiform elements, **characterised in that** it includes a plurality of threads, each of which passes through it at points that are relatively close thereto and which are secured to it by its mid area, each thread having two ends that will be situated outside the vaginal and cutaneous suture lines after the intervention in order to tighten/loosen the mesh.

2. Mesh for the surgical treatment of urinary incontinence, according to claim 1, **characterised in that** said threads pass through pairs of holes mechanically made in the body of the mesh.

3. Mesh for the surgical treatment of urinary incontinence, according to claim 1, **characterised in that** said threads pass through the gaps in the fabric of the mesh itself.
